(19) Europäisches Patentamt
European Patent Office
Office européen des brevets

(11) EP 3 944 864 A1

(12) EUROPEAN PATENT APPLICATION

(43) Date of publication:
**02.02.2022 Bulletin 2022/05**

(21) Application number: **20189034.0**

(22) Date of filing: **31.07.2020**

(51) International Patent Classification (IPC):
***A61K 41/00*** *(2020.01)* ***A61P 35/00*** *(2006.01)*

(52) Cooperative Patent Classification (CPC):
**A61K 41/009; A61K 41/0095**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Theranosticentre S.r.l.**
  **20122 Milano (IT)**
- **Kingston University Higher Education Corporation**
  **Kingston Upon Thames, Surrey KT1 1LQ (GB)**

(72) Inventors:
- **MARTELLINI, Maurizio**
  **20142 Milano (IT)**
- **GHERARDI, Giuseppe**
  **40126 Bologna (IT)**
- **CALABRESE, Gianpiero**
  **Hampton Wick, KT1 4BA (GB)**
- **CAPRIFICO, Anna**
  **Surbiton, KT6 (GB)**
- **BUONOCORE, Federico**
  **84098 Pontecagnano Faiano SA (IT)**

(74) Representative: **Modiano, Micaela Nadia et al**
**Modiano & Partners**
**Via Meravigli, 16**
**20123 Milano (IT)**

# (54) POLYMERS AND HYDROGELS FOR APPLICATION WITH NEUTRON INTRA-OPERATIVE RADIATION THERAPY

(57) The present invention relates to novel functionalised chitosans and hydrogel comprising such functionalised chitosans. The hydrogels of the invention and the pharmaceutical compositions comprising such hydrogels are particularly useful in the Neutron Capture Therapy (NCT) of solid tumours. Also disclosed is a method of preparing the functionalised chitosans according to the invention.

EP 3 944 864 A1

## Description

The present invention relates to polymers and hydrogels comprising such polymers, particularly for use in neutron intra-operative radiation therapy in the Neutron Capture Therapy (NCT) of solid tumours.

NCT is a non-invasive therapeutic procedure for treating locally malignant tumours such as primary tumours arising from the central nervous system (such as brain tumours and spinal tumours) and recurrent head and neck cancers. It is a two step therapeutic procedure: in a first step, a compound containing a non-radioactive isotope having a high propensity to capture slow neutrons is administered to the patient and in the second step, the tumour is irradiated with a thermal neutron beam. The neutrons are captured by the isotope nuclei of the compound previously delivered to and up-taken by the tumour cells and a nuclear fission reaction occurs, the products of which destroy the tumour cells.

The compound containing a non-radioactive isotope used for the procedure has no cytotoxic activity by itself and is designed so that it specifically localizes at the tumour site. Sometimes, the compound can also chelate a radionuclide (also referred to as "radioactive tracer", or "contrast agent"), like $^{68}$Ga or $^{64}$Cu. The presence of a chelated radionuclide is advantageous as it allows to monitor the biodistribution of the compound by means of an imaging technique, such as for example Positron Emission Tomography (PET) imaging.

It has been observed that certain nuclides have the capability to absorb neutrons, the energy of which is below a threshold causing damages to living tissues. The probability of capturing a neutron is determined by the neutron-capture cross section ($\sigma_{th}$) of such nuclides. Consequently, high ($\sigma_{th}$ hnucleotides ($^{10}$B, $^{113}$Cd, $^{235}$U and $^{157}$Gd) have been considered for applications in the NCT of cancers. Among these, $^{10}$B is widely regarded as the most promising nuclide for clinical applications since, on absorption of thermal neutrons, it generates living-tissue-destroying daughter nuclear fragments ($^4He^{2+}$ or $\alpha$ particles and $^7Li^{3+}$ nuclei) that exhibit high Linear Energy Transfer (LET) and short path lengths (< 10 $\mu$m). Thus, Boron Neutron Capture Therapy (BNCT) involves the selective delivery of $^{10}$B-rich agents to tumour cells. These are then irradiated with low-energy neutrons to induce a nuclear reaction that causes the selective destruction of the boron-rich cells (Calabrese et al, (2012), The formulation of polyhedral boranes for the boron neutron capture therapy of cancer, Drug Discovery Today, 17(3-4), 153-159). Vascular damage caused by the non-selective uptake of boron has been the main limiting factor in the application of BNCT with only two agents, *para*-boronophenylalanine (BPA) and borocaptate sodium (BSH), showing sufficient promise for evaluation in the clinic. Although developments in the synthetic chemistry of polyhedral boranes have addressed issues (especially of toxicity to a considerable extent), the optimization of their delivery systems is a challenge that can only be addressed by the development of innovative formulation strategies (Calabrese et al. 2012).

Intra-operative radiotherapy (IORT) is a therapeutic technique, in particular for the treatment of tumours, that associates radiotherapy with surgical intervention. This technique involves the administration of a dose of radiation directly on the tumour to eliminate residual cancer cells which are non-removable by surgical intervention or, after having removed the neoplastic mass, directly on the tumour bed. The administration of the required dose of radiation can preferably be obtained via a device specifically designed for intra-operative radiotherapy as described in European patent application No. EP3522177A, such as the NEUTRON BRUSH® device.

The general emerging scenario for tumour targeting is extending the focus of new research activities. These include not only the design, synthesis and evaluation of more selective tumour-targeting agents (Azab et al, (2005), Boron neutron capture therapy, Studies in Inorganic Chemistry, 22, 337), but also, most importantly, the optimization of their delivery (Calabrese *et al,* 2012), the discovery of new selective targets and the improvement of neutron beam characteristics (Wu et al, (2006), Boron containing macromolecules and nanovehicles as delivery agents for neutron capture therapy, Anti-Cancer Agents in Medicinal Chemistry (Formerly Current Medicinal Chemistry-Anti-Cancer Agents), 6(2), 167-184).

Another direction has been the covalent link of a high affinity compound for targeted BNCT with a radionuclide chelating group as in European patent No. EP3468978B1. The high metabolic activity and correspondingly high membrane potential of mitochondria of neoplastic cells opens up the possibility of targeting cellular organelles, such as mitochondria, and offers the distinct advantage that both non-dividing and dividing cancer cells become accessible to the chemotherapeutic agent (Don et al., (2004), Mitochondria as cancer drug targets, Trends in molecular medicine, 10(8), 372-378). Neoplastic cells can be selectively targeted because they show a high negatively charged mitochondrial transmembrane potential compared to healthy cells. Mitochondria of tumour cells need to meet the metabolic demands of the cells by altering their bioenergetic and biosynthetic activities. Hence, mitochondria increase the rate of glycolysis and metabolism in order to provide nucleotides, lipids and amino acids for the synthesis of macromolecules along with ATP and NADPH, needed for cell proliferation (Weinberg and Chandel, (2015),Targeting mitochondria metabolism for cancer therapy, Nature Chemical Biology, 11(1), pp. 9-15). This difference can be targeted by using delocalized lipophilic cations (DLCs). The accumulation of DLCs driven by the mitochondrial membrane potential may be described by the Nernst equation:

$$\Delta\Psi\ (mV) = 61.5\ \log\ \{[cation]_{in}/[cation]_{out}\}.$$

Because of the approximately 60 mV difference between the mitochondrial transmembrane potential of healthy and tumour cells, the DLC structure tags selectively cancer cells due to its resonance-stabilised positive charge. It has been estimated that 90-95% of intracellular cations are localized in mitochondria. These features lead DLCs to accumulate in the mitochondrial matrix of tumour cells (Murphy, (1997), Selective targeting of bioactive compounds to mitochondria, Trends in biotechnology, 15(8), 326-330). The present inventors previously developed (Calabrese et al, (2008), Carborane-based derivatives of delocalised lipophilic cations for boron neutron capture therapy: synthesis and preliminary in vitro evaluation, Journal of Materials Chemistry, 18(40), 4864-4871) a series of compounds in which carboranes are combined with DLCs. Preliminary *in vitro* evaluation indicated the combined propensity of these agents to target tumour cells and deliver therapeutic relevant quantities of boron, irrespectively of the presence of a covalent bond between the carborane and DLC moieties (Calabrese *et al,* 2008). Hence, it appears that a covalent link between the DLC and the boron moiety may not be necessary for the selective delivery of boron to tumour sites (Ioppolo et al, (2009), Water-soluble phosphonium salts containing 1, 12-dicarba-closo-dodecaborane (12), Tetrahedron Letters, 50(47), 6457-6461). Moreover, insights on the molecular mechanisms have shown DLC-carboranes activate the p53/p21 pathway (Tseligka et al., (2016), Pharmacological development of target-specific delocalized lipophilic cation-functionalized carboranes for cancer therapy, Pharmaceutical research, 33(8), 1945-1958).

The primary approach to the treatment of solid tumours is surgical resection, which alleviates tissue compression and allows a longer survival rate (Schiapparelli et al, (2020), Self-assembling and self-formulating prodrug hydrogelator extends survival in a glioblastoma resection and recurrence model, Journal of Controlled Release, 319, 311-321). Following surgical removal of the solid tumour, an uneven surface of tissue is left behind, which due to tumours cells heterogeneity is characterised by the presence of cancerous stem cells or tumour-initiating cells (Yan et al, (2016), An intriguing target for killing tumour-initiating cells, Vol. 26, Mitochondrion. Elsevier B.V. p. 86-93), unremovable by the surgical operation. However, the removal of these cells is a key step in preventing recurrence. Indeed, these cells are responsible for the infiltrative growth of tumour in the surrounding healthy tissue and are the main cause of tumour recurrence after maximal resection (Yan et al, 2016). The currently adopted chemotherapeutic modalities to eradicate residual tumour-initiating cells induce significant side effects, such as toxicity and unfavourable biodistribution of the chemotherapeutic agent, hence targeted drug delivery is a growingly relevant area for the treatment of tumours (Raave et al, (2018), Chemotherapeutic drug delivery by tumoral extracellular matrix targeting, Vol. 274, Journal of Controlled Release. Elsevier B.V.;. p. 1-8).

An emerging, effective, and promising therapy that can be applied immediately after surgery makes use of injectable biocompatible hydrogels. Injectable hydrogels are promising candidates for *in vivo* minimally invasive surgery (Piantanida et al, (2019), Design of Nanocomposite Injectable Hydrogels for Minimally Invasive Surgery, Accounts of Chemical Research. 52(8):2101-12). This strategy offers several advantages. For instance, injectable hydrogels can fill the cavity just after tumour resection and encapsulate the drug, providing its sustained release (Chakroun et al, (2018), Nanotherapeutic systems for local treatment of brain tumors, Wiley Interdiscip Rev Nanomedicine Nanobiotechnology. 10(1):e1479; Schiapparelli *et al,* 2020). Moreover, hydrogels are constituted by a 3D hydrophilic, non-toxic, biocompatible, polymeric network, absorbing water, and establishing covalent or non-covalent interactions, that confer stability and rigidity, preventing the hydrogel dissolution in water (Fan et al, (2019), Injectable Hydrogels for Localized Cancer Therapy, Vol. 7, Frontiers in Chemistry. 7, 675).

The swelling features of the hydrogel play a key role in its use. For instance, the ratio between the weight of the swollen hydrogel over the dry hydrogel impacts on the solute diffusion coefficient as well as the optical and mechanical features. Limitations of the injectable hydrogels currently used include the low viscosity of the hydrogel precursor solution which needs to be applied through the pressure of a syringe (Piantanida *et al,* 2019). Moreover, after the injection, the material should ideally transform into a gel very quickly, by either physical or chemical sol-gel transitions, to prevent spreading in the surrounding healthy tissue.

One example of injectable hydrogel as a fluorescence surgical marker has been reported by mixing sodium alginate with a mix of indocyanine green and human serum albumin (Sook et al, (2020) Indocyanine green-loaded injectable alginate hydrogel as a marker for precision cancer surgery. Quantitative Imaging in Medicine and Surgery. 10(3), 779).

An alternative has been reported (de la Puente et al (2018), Injectable hydrogels for localized chemotherapy and radiotherapy in brain tumors, Journal of pharmaceutical sciences, 107(3), 922-933) that involves an injectable chitosan hydrogel for the localized delivery of Temozolomide and $^{131}I$ a type of radiotherapy agent. However, the authors reported the active ingredient as incorporated in alginate nanocarriers which means no release over a prolonged period of time. Additionally, these delivery systems also come with their own challenges such as the possibility of dose dumping (Dominguez-Robles et al, (2018), Implantable Polymeric Drug Delivery Devices: Classification, Manufacture, Materials, and Clinical Applications, Polymers, 10(12), 1379), which for both chemotherapeutic and radiotherapy agents can cause

serious side effects to patients.

In view of the above, the aim of the present invention is to provide polymers useful for manufacturing an improved drug delivery system, such as a hydrogel drug delivery system, for application in NCT of solid tumours.

Another object of the present invention is to provide a drug delivery system compatible with pharmacophores used in NCT, particularly in the neutron intra-operative radiation therapy (nIORT®).

Another object of the present invention is to provide a drug delivery system which can be used to enhance the therapeutic dose of a neutron intra-operative therapy device, such as the NEUTRON BRUSH® device.

Another object of the invention is to provide a drug delivery system that is biocompatible and safe to use.

The aim, as well as these and other objects which will become better apparent hereinafter, are achieved by a functionalised chitosan of general formula 1:

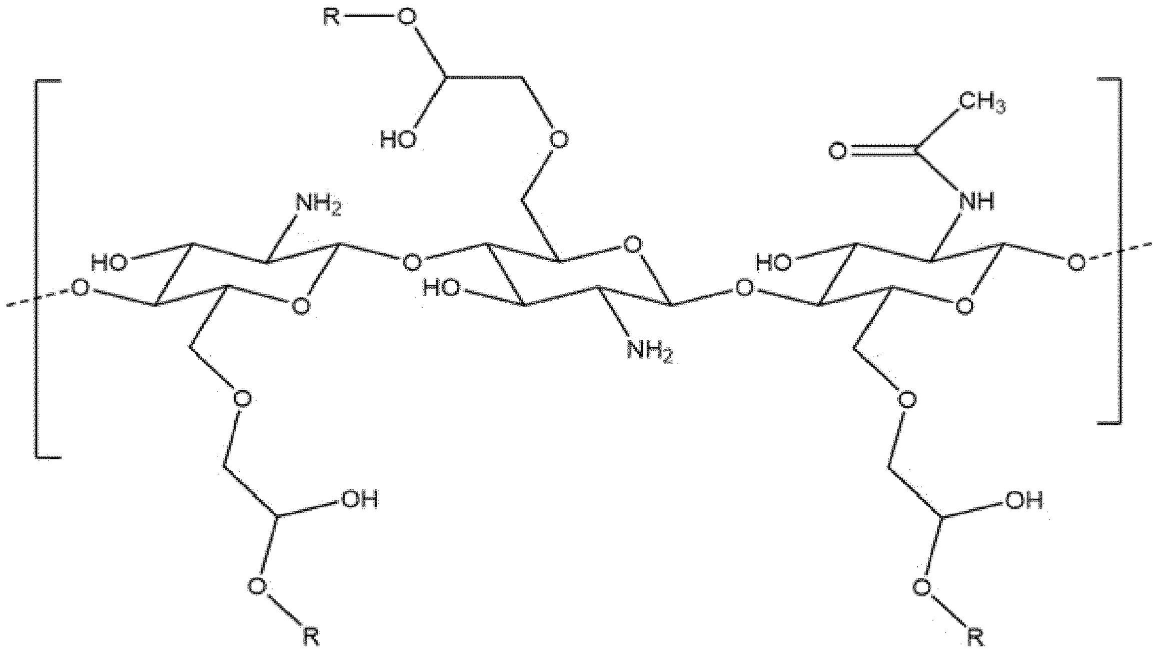

FORMULA 1

wherein R is selected from the group consisting of linear or branched $C_1$-$C_{12}$ alkyl, linear or branched $C_2$-$C_6$ alkenyl and linear or branched $C_2$-$C_6$ alkinyl.

The aim and objects of the present invention are also achieved by a method of preparing the functionalised chitosan according to the invention, said method comprising the steps of:

i) dispersing a chitosan in a 15%-20% (weight/volume) aqueous solution of a base so as to obtain a dispersion with a molar ratio of chitosan/base comprised between 2 and 4;

ii) storing the dispersion at a temperature comprised between -15°C and -25°C overnight, thereby obtaining a frozen dispersion;

iii) allowing the frozen dispersion to thaw at room temperature and adding isopropyl alcohol to the thawed dispersion until a solution with a molar ratio of chitosan/isopropyl alcohol comprised between 5 and 20 is obtained;

iv) stirring the solution obtained in step iii) under reflux conditions at a temperature comprised between 40°C and 50°C;

v) adding to the stirred solution of step iv) an epoxide selected from the group consisting of allyl glycidyl ether, glycidyl methyl ether, ethyl glycidyl ether, glycidyl propargyl ether, glycidyl isopropyl ether, *n*-butyl glycidyl ether, *tert*-butyl glycidyl ether, 2-ethylhexyl glycidyl ether, glycidyl hexadecyl ether and glycidyl lauryl ether until a solution with a molar ratio of chitosan/epoxide comprised between 5 and 20 is obtained;

vi) stirring the solution obtained in step v) under reflux conditions at a temperature comprised between 60°C and 80°C;

vii) allowing the solution obtained in step vi) to cool at room temperature and adding drop-wise an aqueous solution of HCl 0.5M until pH 7 is reached.

The aim and objects of the present invention are also achieved by a hydrogel comprising the functionalised chitosan according to the invention.

The aim and objects of the present invention are also achieved by a pharmaceutical composition comprising

the hydrogel of the invention.

The aim and objects of the invention are achieved also by a hydrogel or a pharmaceutical composition according to the invention for a use selected from the group consisting of diagnosing a tumour, treating a tumour, and diagnosing and treating at the same time a tumour.

Finally the aim and objects of the invention are achieved by a membrane comprising the functionalised chitosan according to the invention.

Further characteristics and advantages of the invention will become better apparent from the following detailed description of the invention.

Controlled drug delivery systems such as hydrogels offer the possibility to increase the bioavailability of a therapeutic agent, hence improving its ability to reach the target tissue at a given dose while minimizing off-targeted side effects (Ahmadi et al, (2015), Chitosan based hydrogels: characteristics and pharmaceutical applications, Research in pharmaceutical sciences, 10(1), 1).

The present invention thus provides novel alkylglycerol-functionalised chitosan polymers and a series of well-characterised hydrogels comprising cross-linked, alkylglycerol-functionalised chitosan polymers as the main matrix component and having improved drug delivery properties.

In a first aspect, the present invention provides a functionalised chitosan of general formula 1:

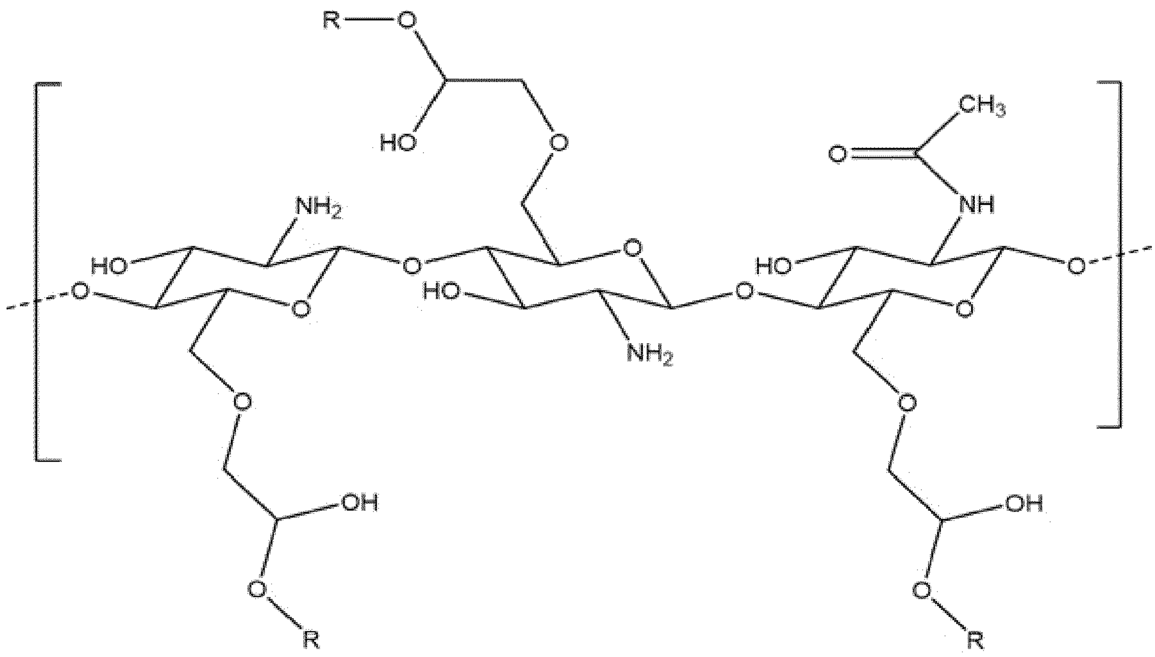

FORMULA 1

wherein R is selected from the group consisting of linear or branched $C_1$-$C_{12}$ alkyl, linear or branched $C_2$-$C_6$ alkenyl and linear or branched $C_2$-$C_6$ alkinyl.

In an embodiment of the functionalised chitosan of the invention, R is selected from the group consisting of methyl, ethyl, allyl, propargyl, isopropyl, *n*-butyl, *tert*-butyl, 2-ethylhexyl, hexadecyl and lauryl. Preferably, R is selected from the group consisting of methyl, allyl, isopropyl, n-butyl and *tert*-butyl.

In a more preferred embodiment, R is methyl.

In another more preferred embodiment, R is ethyl.

In another more preferred embodiment, R is allyl.

In another more preferred embodiment, R is propargyl.

In another more preferred embodiment, R is isopropyl.

In another more preferred embodiment, R is *n*-butyl.

In another more preferred embodiment, R is *tert*-butyl.

In another more preferred embodiment, R is 2-ethylhexyl.

In another more preferred embodiment, R is hexadecyl.

In yet another more preferred embodiment, R is lauryl.

In a second aspect, the present invention provides a method of preparing the functionalised chitosan according to the invention, said method comprising the steps i) to vii) as defined above.

In an embodiment of the method, the base in step i) is selected from the group consisting of hydroxides of alkali metals and hydroxides of alkaline earth metals. Non limiting examples of bases that can be used in step i) of the method of the invention comprise NaOH and KOH.

Preferably, refluxing in step iv) is conducted while stirring at a speed comprised between 200 and 300 rpm preferably for 1 to 3h, more preferably refluxing in step iv) is conducted while stirring at a speed comprised between 200 and 300 rpm for 1 to 3h.

Preferably, refluxing in step vi) is conducted while stirring at a speed comprised between 200 and 300 rpm preferably for 2 to 8h, more preferably refluxing in step vi) is conducted while stirring at a speed comprised between 200 and 300 rpm for 2 to 8h.

Preferably, the chitosan has a molecular weight comprised between 50,000 and 190,000 Da as determined by viscometry.

A common method for molecular weight (MW) determination is viscometry, where the target property connected to the polymer viscosity average MW ($M_v$) is its intrinsic viscosity ([η]), according to the Mark-Houwink equation (1):

$$[\eta]=KM_v^{\alpha} \quad (1)$$

Indeed, upon dissolution of a polymer into a liquid, the two components (water and polymer) interact with each other, so that the polymer's dimension increases along with the viscosity of the solvent. Therefore, [η] relates to the intrinsic ability of the polymer to increase the viscosity of the solvent at a given temperature.

In the Mark-Houwink equation, K and α are constant properties of a given polymer/solvent/temperature system.

The K values for flexible chains are in the range of $10^{-3}$ to $10^{-1}$ mL/g.

The α parameter can be related to the gross solution conformation of the polymer by using the "Haug's triangle" representation of conformations (Errington et al. (1993), Hydrodynamic characterization of chitosans varying in degree of acetylation, International journal of biological macromolecules, 15(2), 113-117 ): α is = 0 for a compact sphere; α is between 0.5-0.8 for a random coil; α is >1 for a rigid cod.

In the case of chitosan dissolved in a solvent (e.g. 1 wt.% in 1% acetic acid, at 25°C), α is > 0.7, since the polymer chains are in a random coil.

An experimental protocol for MW determination is as follows.

Chitosan ($7.5\times10^{-3}$ g/mL) is dissolved in sodium acetate buffer (pH 4.93; 0.3 M AcOH, d = 1.006 g/mL) according to Rinaudo et al. (1993), Characterization of chitosan, Influence of ionic strength and degree of acetylation on chain expansion, International journal of biological macromolecules, 15(5), 281-285, and the viscosity of each solution is measured using an Ostwald U-tube viscometer:
The U-tube is filled up with chitosan solution until the mark on the glass of the lower bulb (line C).

The system is equilibrated in a thermostatically controlled water bath maintained at 25°C for 5 min.

The solution is passed on the upper bulb (line A) of the capillary viscometer by suction.

The flow time (seconds) of the same volume of solution passing from the upper bulb (line A up to line B) is controlled by the thin capillary underneath and recorded.

The flow time data (300 s) are used to calculate the [η] of the solution.

For example, a chitosan having a [η] calculated of 500 ml/g has a viscosity average MW of 100 KDa.

In a third aspect, the present invention provides a hydrogel comprising a functionalised chitosan according to the invention. In the hydrogel according to the invention the functionalised chitosan is in a cross-linked form.

The hydrogel according to the invention can be loaded with different pharmacophores or nanostructures that can favourably interact with neutrons in the NCT of cancers. These pharmacophores or nanostructures refer to chemical entities, which directly or indirectly facilitate the neutron capture event and present any of the following elements: Li, B, Cd, Cu, Am, Sm, Gd, Hg, Au, in individual molecular compounds (pharmacophores) as well as in nanostructured or supramolecular arrangements of the same elements, including polyhedral boranes, poly boronic acids, boron nitride nanotubes, B nanoparticles, Cu nanoparticles or Au nanoparticles.

In a preferred embodiment, the hydrogel according to the invention comprises a selective NCT pharmacophore selected from the group consisting of:

i) a compound of general formula 2

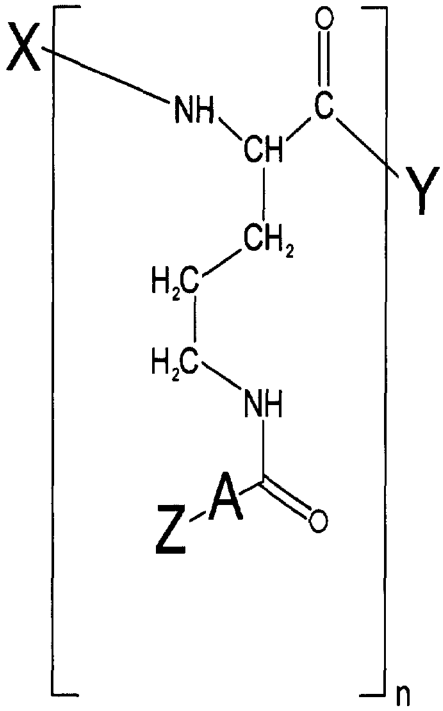

FORMULA 2

wherein

X is a radionuclide chelating group;
Y is a peptide;
A is selected from the group consisting of a bond, an alkylene group and a carboxyl group;
Z is selected from the group consisting of an optionally substituted carborane group and an optionally substituted dodecarborate group;
n is an integer from 1 to 11;and

ii) a compound of general formula 3

C-D-B FORMULA 3

wherein

D is an alkyl linker;
B is a peptide or an optionally modified delocalized lipophilic cation (DLC);
C is an element selected from the group consisting of Li, B, Cd, Cu, Am, Sm, Gd, Hg, and Au, in individual molecular compounds as well as in nanostructured or supramolecular arrangements of the same elements, including polyhedral boranes, poly boronic acids, boron nitride nanotubes, B nanoparticles, Cu nanoparticles or Au nanoparticles;

The hydrogels of the present invention are prepared according to known methods, such as for example according to the disclosure of Azab et al. (2006), Crosslinked chitosan implants as potential degradable devices for brachytherapy: In vitro and in vivo analysis, Journal of Control Release; 111(3):281-9.
In a fourth aspect, the present invention refers to a pharmaceutical composition comprising the hydrogel of the invention.
In one embodiment, such pharmaceutical composition can be in injectable form.
In another embodiment, the pharmaceutical composition can further comprise one or more radionuclides (radioactive tracers). Examples of such radionuclides can be, e.g., $^{68}Ga$ or $^{64}Cu$. Radionuclides are bound by a chelating group of the pharmacophore, for example the chelating group (X) of the compound of formula 2, thus allowing the compound distribution at the tumour site to be traced by imaging (e.g. PET) techniques.

Alternatively, when such imaging is desired, a radionuclide-containing formulation can be administered separately to the patient, at the same time of, or shortly before, or shortly after administration of the hydrogel of the present invention, so that chelation of the radionuclides by this compound occurs.

In another preferred embodiment, the pharmaceutical composition can further comprise one or more pharmaceutical excipients, which are well-known to a person skilled in the field of pharmaceutical formulations and in particular of compositions for BNCT.

In a fifth aspect, the present invention refers to a hydrogel or a pharmaceutical composition according to the invention for a use selected from the group consisting of diagnosing a tumour, treating a tumour, and diagnosing and treating at the same time a tumour in a patient.

In a preferred embodiment, the hydrogel for the use according to the invention is in combination with BNCT.

In another preferred embodiment, the hydrogel for the use according to the invention is in combination with an imaging technique, preferably Positron Emission Tomography (PET) imaging.

Within this fifth aspect, the present invention also provides a method for diagnosing a tumour, treating a tumour, or diagnosing and at the same time treating a tumour by administering the hydrogel or the pharmaceutical composition according to the present invention to a patient having a tumour.

In a sixth aspect, the present invention refers to an article comprising the functionalised chitosan of the invention, wherein the article is preferably a membrane (film).

A membrane is obtained by first combining a hydrogel of the present invention with a plasticiser to obtain a suspension and then casting and drying the obtained suspension on a glass plate. The residual plasticiser is removed by washing the membranes with deionised water. Non limiting examples of suitable plasticisers are those selected from the group consisting of 1-ethyl-3-methylimidazolium acetate, glycerol, ethylene glycol, poly(ethylene glycol), and propylene glycol. A preferred plasticiser is 1-ethyl-3-methylimidazolium acetate.

The invention will now be further described by way of the following non limiting examples:

## EXAMPLE 1: PREPARATION OF FUNCTIONALISED CHITOSAN

Chitosan (3 g) having a low MW between 50,000 and 190,000 Da (Sigma-Aldrich) was dispersed in a solution of NaOH (15% w/v).

The resulting chitosan solution was then stored at -20°C overnight to allow alkalinisation.

After thawing at room temperature, 30 ml of isopropyl alcohol were added to the solution and the mixture was refluxed at 40 °C, under stirring at 250 rpm for 2 h.

After 2 h, 30 ml of *n*-butyl glycidyl ether were added to the mixture, which was then refluxed at 60 °C for 2 h.

After allowing the mixture to cool to room temperature, a solution of 0.5 M HCl was added drop-wise until pH = 7 was reached.

The mixture was then purified by washing with water (to remove NaCl) and with acetone (to remove unreacted epoxide).

## EXAMPLE 2: PREPARATION OF A NON-LOADED HYDROGEL

A solution of 100 mL 0.1 M acetic acid containing 1 g of functionalised chitosan prepared according to Example 1 was stirred until dissolution of all the functionalised chitosan. Subsequently a solution of glutaraldehyde 0.2% w/v was added to the solution containing the functionalised chitosan and formation of a viscous gel was observed. The gel was then left to stabilize for 5 hours. The obtained hydrogel was characterized by Fourier Transform Infrared Spectroscopy (FTIR) and shear-stress technique.

## EXAMPLE 3: PREPARATION OF A LOADED HYDROGEL

A solution of 100 mL 0.1 M acetic acid containing 1 g of functionalised chitosan prepared according to Example 1 was stirred until dissolution of all the functionalised chitosan. Subsequently, a solution of glutaraldehyde 0.2% w/v and methyl *ortho*-carboranyl triphenyl phosphonium methylene bromide was added to the solution containing the functionalised chitosan and formation of a viscous gel was observed. The gel was then left to stabilize for 5 hours. The obtained hydrogel loaded with the carborane derivative was characterized by Fourier Transform Infrared Spectroscopy (FTIR) and shear-stress technique.

## EXAMPLE 4: PREPARATION OF A MEMBRANE

The plasticiser 1-ethyl-3-methylimidazolium acetate (1% w/v) was added to a hydrogel prepared according to Examples 2 or 3 to obtain a suspension. The resulting suspension was then cast on a glass plate, and dried at 60 °C

for 2.5 h to obtain a membrane. The residual plasticiser was removed by washing with deionized water and the membrane was stored in a desiccator until use.

In practice it has been found that the functionalised chistosan polymer and hydrogel according to the invention fully achieve the intended aim and objects, since they allow manufacturing of an improved, biocompatible and safe to use, drug delivery system for application in NCT of solid tumours, which is compatible with pharmacophores used in NCT, particularly in the neutron intra-operative radiation therapy (nIORT®).

## Claims

1. A functionalised chitosan of general formula 1:

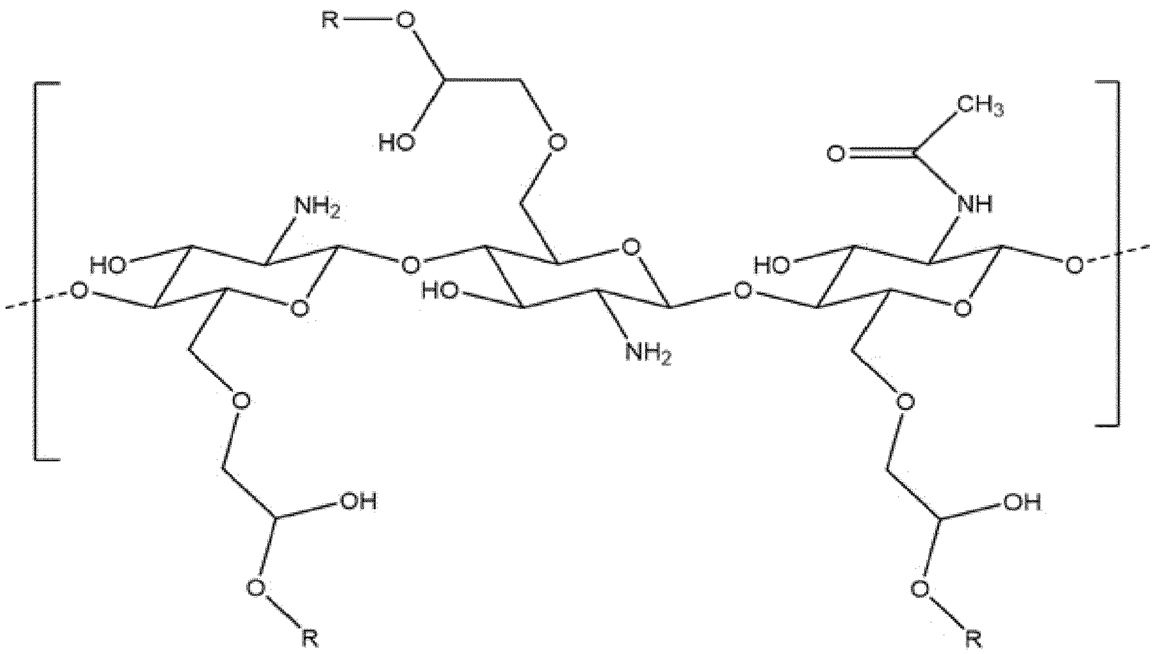

FORMULA 1

wherein R is selected from the group consisting of linear or branched $C_1$-$C_{12}$ alkyl, linear or branched $C_2$-$C_6$ alkenyl and linear or branched $C_2$-$C_6$ alkinyl.

2. The functionalised chitosan of claim 1 wherein R is selected from the group consisting of methyl, allyl, isopropyl, *n*-butyl and *tert*-butyl.

3. A method of preparing the functionalised chitosan according to claim 1 or 2, said method comprising the steps of:

i) dispersing a chitosan in a 15%-20% (weight/volume) aqueous solution of a base so as to obtain a dispersion with a molar ratio of chitosan/base comprised between 2 and 4;
ii) storing the dispersion at a temperature comprised between -15°C and -25°C overnight, thereby obtaining a frozen dispersion;
iii) allowing the frozen dispersion to thaw at room temperature and adding isopropyl alcohol to the thawed dispersion until a solution with a molar ratio of chitosan/isopropyl alcohol comprised between 5 and 20 is obtained;
iv) stirring the solution obtained in step iii) under reflux conditions at a temperature comprised between 40°C and 50°C;
v) adding to the stirred solution of step iv) an epoxide selected from the group consisting of allyl glycidyl ether, glycidyl methyl ether, ethyl glycidyl ether, glycidyl propargyl ether, glycidyl isopropyl ether, n-butyl glycidyl ether, tert-butyl glycidyl ether, 2-ethylhexyl glycidyl ether, glycidyl hexadecyl ether and glycidyl lauryl ether until a solution with a molar ratio of chitosan/epoxide comprised between 5 and 20 is obtained;
vi) stirring the solution obtained in step v) under reflux conditions at a temperature comprised between 60°C and 80°C;

vii) allowing the solution obtained in step vi) to cool at room temperature and adding drop-wise an aqueous solution of HCl 0.5M until pH 7 is reached.

**4.** The method of claim 3 wherein the chitosan has a molecular weight comprised between 50,000 and 190,000 Da as determined by viscometry.

**5.** The method of claim 3 or 4 wherein refluxing in step iv) is conducted while stirring at a speed comprised between 200 and 300 rpm preferably for 1 to 3h.

**6.** The method of any one of claims 3 to 5 wherein refluxing in step vi) is conducted while stirring at a speed comprised between 200 and 300 rpm preferably for 2 to 8h.

**7.** A hydrogel comprising the functionalised chitosan of claim 1 or 2.

**8.** The hydrogel according to claim 7 further comprising a selective neutron capture therapy (NCT) pharmacophore.

**9.** The hydrogel according to claim 8 wherein said selective (NCT) pharmacophore is selected from the group consisting of:

i) a compound of general formula 2

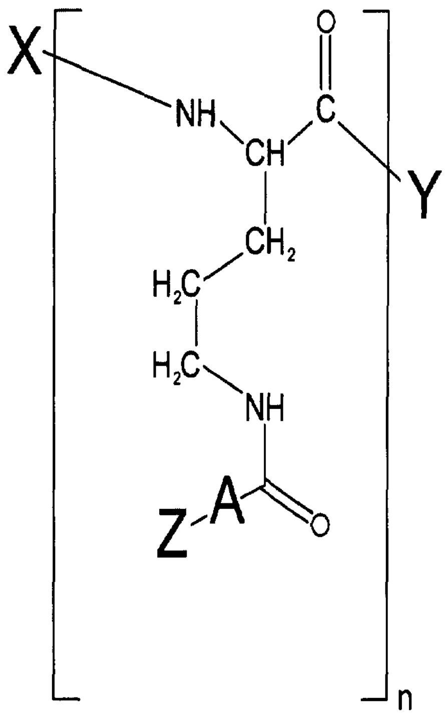

FORMULA 2

wherein

X is a radionuclide chelating group;
Y is a peptide;
A is selected from the group consisting of a bond, an alkylene group and a carboxyl group;
Z is selected from the group consisting of an optionally substituted carborane group and an optionally substituted dodecarborate group;
n is an integer from 1 to 11; and

ii) a compound of general formula 3

C-D-B FORMULA 3

wherein

D is an alkyl linker;
B is a peptide or an optionally modified delocalized lipophilic cation (DLC);
C is an element selected from the group consisting of Li, B, Cd, Cu, Am, Sm, Gd, Hg, and Au, in individual molecular compounds as well as in nanostructured or supramolecular arrangements of the same elements, including polyhedral boranes, poly boronic acids, boron nitride nanotubes, B nanoparticles, Cu nanoparticles or Au nanoparticles.

**10.** A pharmaceutical composition comprising the hydrogel of anyone of claims 7 to 9.

**11.** The hydrogel of anyone of claims 7 to 9 or the pharmaceutical composition according to claim 10 for a use selected from the group consisting of diagnosing a tumour, treating a tumour, and diagnosing and treating at the same time a tumour in a patient in need thereof.

**12.** The hydrogel or pharmaceutical composition for the use according to claim 11 in combination with Neutron Capture Therapy (NCT) of solid tumours.

**13.** The hydrogel or pharmaceutical composition for the use according to claim 11 in combination with an imaging technique, preferably Positron Emission Tomography (PET) imaging.

**14.** A membrane comprising the functionalised chitosan of claim 1 or 2.

Europäisches Patentamt
European Patent Office
Office européen des brevets

# EUROPEAN SEARCH REPORT

Application Number
EP 20 18 9034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/022655 A1 (BUZATU DAN A [US] ET AL) 22 January 2009 (2009-01-22) * claims 1, 7 * ----- | 1-14 | INV. A61K41/00 A61P35/00 |
| A | WO 2017/216816 A1 (THERANOSTICENTRE S R L [IT]) 21 December 2017 (2017-12-21) * claims 1, 10, 17 * ----- | 1-14 | |
| A | WO 2009/056602 A1 (VISCOGEL AB [SE]; ANDERSSON MATS [SE]) 7 May 2009 (2009-05-07) ----- | 1-14 | |
| A | BALAKUMAR VIJAYAKRISHNAN ET AL: "MSof the six isomers of (GlcN)(GlcNAc)aminoglucan tetrasaccharides (diacetylchitotetraoses): Rules of fragmentation for the sodiated molecules and application to sequence analysis of hetero-chitooligosaccharides", CARBOHYDRATE POLYMERS, APPLIED SCIENCE PUBLISHERS, LTD. BARKING, GB, vol. 84, no. 2, 22 April 2010 (2010-04-22), pages 713-726, XP028359683, ISSN: 0144-8617, DOI: 10.1016/J.CARBPOL.2010.04.041 [retrieved on 2010-04-29] ----- | 1-14 | TECHNICAL FIELDS SEARCHED (IPC) |
| | | | A61K |
| A | KATSUAKI HIRANO ET AL: "Classification of Chitosanases by Hydrolytic Specificity toward N 1 , N 4 -Diacetylchitohexaose", BIOSCIENCE BIOTECHNOLOGY BIOCHEMISTRY, vol. 76, no. 10, 23 October 2012 (2012-10-23), pages 1932-1937, XP055760173, JP ISSN: 0916-8451, DOI: 10.1271/bbb.120408 ----- -/-- | 1-14 | |

The present search report has been drawn up for all claims

1

| Place of search | Date of completion of the search | Examiner |
|---|---|---|
| The Hague | 18 December 2020 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 18 9034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | TSELIGKA EIRINI D ET AL: "Pharmacological Development of Target-Specific Delocalized Lipophilic Cation-Functionalized Carboranes for Cancer Therapy", PHARMACEUTICAL RESEARCH, SPRINGER NEW YORK LLC, US, vol. 33, no. 8, 26 April 2016 (2016-04-26), pages 1945-1958, XP036002062, ISSN: 0724-8741, DOI: 10.1007/S11095-016-1930-4 [retrieved on 2016-04-26] ----- | 1-14 | |
| A,D | RINAUDO M ET AL: "Characterization of chitosan. Influence of ionic strength and degree of acetylation on chain expansion", ACTA PAEDIATRICA. SUPPLEMENT, ELSEVIER BV, NL, vol. 15, no. 5, 1 October 1993 (1993-10-01), pages 281-285, XP023459996, ISSN: 0141-8130, DOI: 10.1016/0141-8130(93)90027-J [retrieved on 1993-10-01] ----- | 1-14 | |
| | | | TECHNICAL FIELDS SEARCHED (IPC) |

The present search report has been drawn up for all claims

1

| Place of search | Date of completion of the search | Examiner |
|---|---|---|
| The Hague | 18 December 2020 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 20 18 9034

This annex lists the patent family members relating to the patent documents cited in the above-mentioned European search report. The members are as contained in the European Patent Office EDP file on
The European Patent Office is in no way liable for these particulars which are merely given for the purpose of information.

18-12-2020

| Patent document cited in search report | Publication date | Patent family member(s) | Publication date |
|---|---|---|---|
| US 2009022655 A1 | 22-01-2009 | US 2009022655 A1 | 22-01-2009 |
| | | US 2011027174 A1 | 03-02-2011 |
| | | US 2011085971 A1 | 14-04-2011 |
| | | US 2011085972 A1 | 14-04-2011 |
| WO 2017216816 A1 | 21-12-2017 | EP 3468978 A1 | 17-04-2019 |
| | | WO 2017216816 A1 | 21-12-2017 |
| WO 2009056602 A1 | 07-05-2009 | AU 2008320877 A1 | 07-05-2009 |
| | | BR PI0817895 A2 | 31-03-2015 |
| | | CA 2704162 A1 | 07-05-2009 |
| | | CN 101903408 A | 01-12-2010 |
| | | EP 2209813 A1 | 28-07-2010 |
| | | JP 5725862 B2 | 27-05-2015 |
| | | JP 2011500955 A | 06-01-2011 |
| | | KR 20100083836 A | 22-07-2010 |
| | | NZ 584996 A | 27-07-2012 |
| | | RU 2010117016 A | 10-12-2011 |
| | | US 2010316715 A1 | 16-12-2010 |
| | | WO 2009056602 A1 | 07-05-2009 |
| | | ZA 201003041 B | 27-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3522177 A
- EP 3468978 B1


### Non-patent literature cited in the description


- **CALABRESE et al.** The formulation of polyhedral boranes for the boron neutron capture therapy of cancer. *Drug Discovery Today,* 2012, vol. 17 (3-4), 153-159
- **AZAB et al.** Boron neutron capture therapy. *Studies in Inorganic Chemistry,* 2005, vol. 22, 337
- **WU et al.** Boron containing macromolecules and nanovehicles as delivery agents for neutron capture therapy. *Anti-Cancer Agents in Medicinal Chemistry (Formerly Current Medicinal Chemistry-Anti-Cancer Agents),* 2006, vol. 6 (2), 167-184
- **DON et al.** Mitochondria as cancer drug targets. *Trends in molecular medicine,* 2004, vol. 10 (8), 372-378
- **WEINBERG ; CHANDEL.** Targeting mitochondria metabolism for cancer therapy. *Nature Chemical Biology,* 2015, vol. 11 (1), 9-15
- **MURPHY.** Selective targeting of bioactive compounds to mitochondria. *Trends in biotechnology,* 1997, vol. 15 (8), 326-330
- **CALABRESE et al.** Carborane-based derivatives of delocalised lipophilic cations for boron neutron capture therapy: synthesis and preliminary in vitro evaluation. *Journal of Materials Chemistry,* 2008, vol. 18 (40), 4864-4871
- **IOPPOLO et al.** Water-soluble phosphonium salts containing 1, 12-dicarba-closo-dodecaborane (12). *Tetrahedron Letters,* 2009, vol. 50 (47), 6457-6461
- **TSELIGKA et al.** Pharmacological development of target-specific delocalized lipophilic cation-functionalized carboranes for cancer therapy. *Pharmaceutical research,* 2016, vol. 33 (8), 1945-1958
- **SCHIAPPARELLI et al.** Self-assembling and self-formulating prodrug hydrogelator extends survival in a glioblastoma resection and recurrence model. *Journal of Controlled Release,* 2020, vol. 319, 311-321
- An intriguing target for killing tumour-initiating cells. **YAN et al.** Mitochondrion. Elsevier B.V, 2016, vol. 26, 86-93
- Chemotherapeutic drug delivery by tumoral extracellular matrix targeting. **RAAVE et al.** Journal of Controlled Release. Elsevier B.V, 2018, vol. 274, 1-8
- **PIANTANIDA et al.** Design of Nanocomposite Injectable Hydrogels for Minimally Invasive Surgery. *Accounts of Chemical Research,* 2019, vol. 52 (8), 2101-12
- Nanotherapeutic systems for local treatment of brain tumors. **CHAKROUN et al.** Interdiscip Rev Nanomedicine Nanobiotechnology. Wiley, 2018, vol. 10, e1479
- **FAN et al.** Injectable Hydrogels for Localized Cancer Therapy. *Frontiers in Chemistry,* 2019, vol. 7 (7), 675
- **SOOK et al.** Indocyanine green-loaded injectable alginate hydrogel as a marker for precision cancer surgery. *Quantitative Imaging in Medicine and Surgery,* 2020, vol. 10 (3), 779
- **DE LA PUENTE et al.** Injectable hydrogels for localized chemotherapy and radiotherapy in brain tumors. *Journal of pharmaceutical sciences,* 2018, vol. 107 (3), 922-933
- **DOMINGUEZ-ROBLES et al.** Implantable Polymeric Drug Delivery Devices: Classification, Manufacture, Materials, and Clinical Applications. *Polymers,* 2018, vol. 10 (12), 1379
- **AHMADI et al.** Chitosan based hydrogels: characteristics and pharmaceutical applications. *Research in pharmaceutical sciences,* 2015, vol. 10 (1), 1
- **ERRINGTON et al.** Hydrodynamic characterization of chitosans varying in degree of acetylation. *International journal of biological macromolecules,* 1993, vol. 15 (2), 113-117
- **RINAUDO et al.** Characterization of chitosan, Influence of ionic strength and degree of acetylation on chain expansion. *International journal of biological macromolecules,* 1993, vol. 15 (5), 281-285
- **AZAB et al.** Crosslinked chitosan implants as potential degradable devices for brachytherapy: In vitro and in vivo analysis. *Journal of Control Release,* 2006, vol. 111 (3), 281-9